# EUROPÄISCHE PATENTSCHRIFT

(11) **EP 2 347 251 B1**
(45) Veröffentlichungstag und Bekanntmachung des Hinweises auf die Patenterteilung: **26.02.2014**
(21) Anmeldenummer: 09744025.9
(22) Anmeldetag: 12.10.2009
(51) Int. Cl.: G01N 33/36, G01N 27/22

(54) **Kapazitive Messschaltung für Garninspektion**
Capacitive measuring circuit for yarn inspection
Circuit de mesure capacitif à l'inspection d'un fil

(30) Priorität: 16.10.2008 CH 16442008
(43) Veröffentlichungstag der Anmeldung: 27.07.2011
(60) Teilanmeldung: 13193263.4
(73) Patentinhaber: Uster Technologies AG, 8610 Uster (CH)
(72) Erfinder: GEHRIG, Reto, 8406 Winterthur (CH); OTT, Philipp, CH-8496 Steg (CH); JOSS, Rolf, CH-8810 Horgen (CH)
(74) Vertreter: Pliska, Pavel
(86) Internationale Anmeldenummer: PCT/CH2009/000327
(87) Internationale Veröffentlichungsnummer: WO 2010/043063

(56) Entgegenhaltungen:
- EP-A1- 0 924 513
- EP-A2- 1 124 134
- DE-A1- 19 535 177
- US-A- 3 757 211

## Beschreibung

Die vorliegende Erfindung liegt auf dem Gebiet der elektrischen Messschaltungen. Sie betrifft eine kapazitive Messschaltung gemäss dem Oberbegriff des ersten Patentanspruchs und ein Verfahren zum Betreiben einer kapazitiven Messschaltung gemäss dem Oberbegriff eines weiteren unabhängigen Patentanspruchs. Die Erfindung erlaubt ein automatisches Abgleichen der Messschaltung, ein Simulieren von Messungen mit der Messschaltung, ein Testen der Messschaltung oder ein Testen von der Messschaltung nachgeschalteten Komponenten.

Ein bevorzugtes Einsatzgebiet für die Erfindung ist die kapazitive Prüfung von länglichen, vorzugsweise textilen Gebilden wie Kardenband, Vorgarn, Garn oder Gewebe. Somit betrifft die Erfindung auch eine Vorrichtung zur kapazitiven Untersuchung eines bewegten länglichen Prüfgutes, gemäss dem Oberbegriff eines weiteren Patentanspruchs. Eine derartige Untersuchung kann bspw. die Detektion von Fremdstoffen, das Erkennen von Änderungen der Masse pro Längeneinheit und/oder die Messung von Feuchtigkeit im Prüfgut zum Ziel haben. Die Erfindung kann bspw. im Produktionsprozess (online) in Garnreinigern auf Spinn- oder Spulmaschinen oder in der Laborprüfung (offline) in Garnprüfgeräten eingesetzt werden.

### STAND DER TECHNIK

Es ist eine Vielzahl verschiedenartiger Vorrichtungen zur Untersuchung oder Prüfung von länglichem textilem Prüfgut wie bspw. Kardenband, Vorgarn, Garn oder Gewebe bekannt. Sie lassen sich nach ihrer Anwendung in die beiden Klassen Laborprüfung (offline) und Prüfung während des Produktionsprozesses (online) einteilen. Die Vorrichtungen verwenden verschiedene bekannte Sensorprinzipien; hier interessiert besonders das kapazitive Messprinzip. Dabei wird eine Messschaltung mit einem Messkondensator, der als Plattenkondensator ausgebildet ist, zur Verfügung gestellt. An die Messschaltung wird eine elektrische Wechselspannung angelegt, wodurch im Messkondensator ein elektrisches Wechselfeld erzeugt wird. Das Prüfgut wird durch den Plattenkondensator hindurch bewegt und dem elektrischen Wechselfeld ausgesetzt. Es werden dielektrische Eigenschaften des Prüfgutes ermittelt. Aus den dielektrischen Eigenschaften werden Parameter des Prüfgutes wie Masse pro Längeneinheit und/oder Materialzusammensetzung bestimmt. Beispiele für geeignete Messschaltungen und Auswerteschaltungen für deren Ausgangssignale finden sich in den Schriften EP-0'924'513 A1, WO-2006/105676 A1 und WO-2007/115416 A1. Eine als Kapazitätsmessbrücke ausgestaltete Messchaltung, beinhaltend den Messkondensator und eine durch ein elektrisches Steuersignal veränderbare Kapazität, ist aus DE 19535177 bekannt.

Um genaue, von äusseren Einflüssen wie Lufttemperatur oder Luftfeuchtigkeit nicht beeinflusste Messungen durchführen zu können, wird häufig eine Kompensationsmethode angewendet. Zu diesem Zweck beinhaltet die Messschaltung nebst dem eigentlichen Messkondensator einen Referenzkondensator. Dieser kann z. B. durch Zufügen einer dritten, parallel zu den beiden Messkondensatorplatten angeordneten und elektrisch in Serie zu ihnen geschalteten Kondensatorplatte gebildet werden.

Ohne Prüfgut sollte die Messschaltung bei angelegter Wechselspannung ein Ausgangssignal Null liefern. In der Praxis genügt es jedoch wegen verschiedener Unvollkommenheiten realer elektrischer Komponenten nicht, die Messschaltung symmetrisch aufzubauen, um ohne Prüfgut ein Nullsignal zu erhalten. Jede einzelne Messschaltung muss zur Symmetrisierung individuell abgeglichen werden. Der Symmetrieabgleich erfolgt in der Produktion durch den Hersteller und bei Bedarf während des Unterhalts durch eine Serviceperson. Zu diesem Zweck wird üblicherweise die Kapazität mindestens eines zur Messschaltung parallel geschalteten Trimmkondensators verändert. Das Trimmen erfolgt manuell mit einem geeigneten Werkzeug, z. B. einem Schraubendreher. Alternativ wird das bekannte Verfahren des Lasertrimmens angewendet. In jedem Fall muss die Vorrichtung für den Abgleich geöffnet werden. Ein solcher manueller Abgleich ist mühsam, zeitaufwändig und kostspielig.

### DARSTELLUNG DER ERFINDUNG

Es ist deshalb eine Aufgabe der vorliegenden Erfindung, eine Messschaltung zu schaffen, welche die obigen Nachteile nicht aufweist. Die Messschaltung soll einfach, schnell, kostengünstig und insbesondere automatisch abgleichbar sein. Ferner soll ein Verfahren zum Betreiben einer erfindungsgemässen Messschaltung angegeben werden. Es soll auch eine kapazitive Vorrichtung zur Untersuchung eines bewegten länglichen Prüfgutes geschaffen werden, welche einfach, schnell, kostengünstig und automatisch abgleichbar ist. Eine weitere Aufgabe der vorliegenden Erfindung ist es, ein Verfahren zum Simulieren von Messungen mit der Messschaltung, zum Testen der Messschaltung oder zum Testen von der Messschaltung nachgeschalteten Komponenten anzugeben.

Diese und andere Aufgaben werden durch die erfindungsgemässe Messschaltung, die erfindungsgemässe Vorrichtung und die erfindungsgemässen Verfahren, wie sie in den unabhängigen Patentansprüchen definiert sind, gelöst. Vorteilhafte Ausführungsformen sind in den abhängigen Patentansprüchen angegeben.

Die Erfindung beruht auf der Idee, die Kapazität mindestens eines Teils der Messschaltung mit einem elektrischen Steuersignal, ohne mechanische oder sonstige Eingriffe, verändern zu können. Dies eröffnet die Möglichkeit eines automatischen Abgleichs der Messschaltung.

Dementsprechend beinhaltet die erfindungsgemässe kapazitive Messschaltung eine auszumessende Kondensatoranordnung und mindestens ein Bauteil mit einer durch mindestens ein elektrisches Steuersignal veränderbare Kapazität.

Unter dem Begriff "Kondensatoranordnung" wird in dieser Schrift eine Anordnung mit zwei Körpern, die vom elektrischen Wechselsignal des Wechselsignalgenerators ungleichartig aufladbar und durch mindestens ein Dielektrikum voneinander getrennt sind. In einer bevorzugten Ausführungsform handelt es sich bei der Kondensatoranordnung um einen Kondensator mit zwei voneinander beabstandeten Platten, zwischen denen sich Luft befindet und zwischen die ein zu untersuchendes bewegtes längliches textiles Prüfgut einführbar ist.

Das mindestens eine Bauteil kann ein Bauteil aus der folgenden Gruppe sein: Kapazitätsdiode, andere Diode, eine Mehrzahl von zueinander parallel geschalteten Abgleichkondensatoren, die mit entsprechenden elektrischen Steuersignalen zu- und wegschaltbar sind, insbesondere MEMS-Kondensatoren, in Sperrrichtung betriebener Transistor, Metall-Oxid-Halbleiter-Feldeffekttransistor, Kondensator, dessen Elektrodenabstand mittels eines Piezoelementes veränderbar ist. Ein oder mehrere Kondensatoren und/oder ein oder mehrere Schalter im mindestens einen Bauteil können vorteilhafterweise als mikro-elektromechanisches System (englisch micro-electro-mechanical system, MEMS) ausgeführt sein. Die Messschaltung kann als Kapazitätsmessbrücke mit mindestens zwei Brückenzweigen ausgebildet sein, wobei einer der Brückenzweige ein Messzweig ist, der die auszumessende Kondensatoranordnung beinhaltet, und mindestens ein anderer der Brückenzweige das mindestens eine Bauteil beinhaltet.

Die erfindungsgemässe Messschaltung wird vorzugsweise zur kapazitiven Untersuchung eines bewegten länglichen textilen Prüfgutes wie Kardenband, Vorgarn, Garn oder Gewebe verwendet, wobei das bewegte Prüfgut die auszumessende Kondensatoranordnung beeinflusst.

Die erfindungsgemässe Vorrichtung zur kapazitiven Untersuchung eines bewegten länglichen Prüfgutes beinhaltet eine erfindungsgemässe kapazitive Messschaltung mit einem Messkondensator zur Aufnahme des Prüfgutes und mindestens einer durch mindestens ein elektrisches Steuersignal veränderbaren Kapazität.

Die erfindungsgemässe Vorrichtung beinhaltet vorzugsweise Mittel zum Anlegen eines Wechselspannungssignals an die Messschaltung zwecks Erzeugung eines elektrischen Wechselfeldes im Messkondensator, und eine Durchgangsöffnung für das Prüfgut im Messkondensator, welche Durchgangsöffnung vom elektrischen Wechselfeld beaufschlagbar ist. Die Messschaltung ist als Kapazitätsmessbrücke mit vier Brückenzweigen ausgebildet, wobei einer der Brückenzweige den Messkondensator beinhaltet, ein anderer der Brückenzweige einen Referenzkondensator beinhaltet und zwei weitere Brückenzweige je eine veränderbare Kapazität aufweisen.

Im erfindungsgemässen Verfahren zum Betreiben einer Messschaltung wird die mindestens eine veränderbare Kapazität durch mindestens ein elektrisches Steuersignal verändert. Während des Abgleichs der erfindungsgemässen Messschaltung wird die auszumessende Kapazität im Wesentlichen zeitlich unverändert belassen. Überhaupt sollten sich die Kapazitäten der Messschaltung, mit Ausnahme der veränderbaren Kapazität, während des Abgleichs nicht ändern. Ein elektrisches Eingangssignal wird an die Messschaltung angelegt. Ein elektrisches Ausgangssignal der Messschaltung wird abgegriffen. Das elektrische Steuersignal wird durch das Ausgangssignal beeinflusst. Die Beeinflussung des elektrischen Steuersignals durch das Ausgangssignal erfolgt vorzugsweise automatisch in einem geschlossenen Regelkreis.

Die Erfindung ist nützlich zum Abgleichen der Messschaltung. Dank der Erfindung wird der Abgleich der Messschaltung einfach, schnell und kostengünstig. Das Gerät muss nicht geöffnet werden, um die Messschaltung abzugleichen. Der Abgleich kann jederzeit erfolgen. Er kann automatisch vom Gerät selbst vorgenommen werden, ohne Eingriff einer Bedienungsperson. Dadurch wird es auch möglich, einen Abgleich zu irgendeinem beliebigen Zeitpunkt durchzuführen. So könnte ein Abgleich vor jeder Messung mit der Messschaltung, vor jeder zehnten Messung, bei wesentlichen Änderungen von Langzeiteigenschaften des Ausgangssignals oder nach einer grösseren Änderung der Umgebungsbedingungen automatisch durchgeführt werden. Dies kann eine Erhöhung der Messgenauigkeit, der Messzuverlässigkeit, der Reproduzierbarkeit und allgemein eine Verbesserung der Messresultate zur Folge haben.

Die veränderbare Kapazität kann aber nicht nur für das Abgleichen der Messschaltung nützlich sein. Sie kann auch zur Einspeisung von beliebigen elektrischen Steuersignalen in die Messschaltung dienen. Mittels solcher Steuersignale wird die Messschaltung gezielt verstimmt. Eine gezielte Verstimmung der Messschaltung kann dieselbe oder eine ähnliche Wirkung haben wie eine Verstimmung durch eine Änderung der auszumessenden Kapazität. Durch Einspeisen von Steuersignalen in die Messschaltung über die veränderbare Kapazität können also Messungen simuliert werden. Dabei sollten sich die Kapazitäten der Messschaltung, mit Ausnahme der veränderbaren Kapazität, nicht ändern. Simulierte Messungen können z. B. zum Testen einer Auswerteschaltung und zur Fehlersuche in der Auswerteschaltung verwendet werden. Sie können einem Abgleich der Auswerteschaltung, z. B. von in der Auswerteschaltung enthaltenen Filtern, dienen. Sie können auch zum Testen und/oder Einstellen einer Vorrichtung, welche die Messschaltung beinhaltet, dienen. Es ist möglich, vorgängig Ausgangssignale realer Messungen mit derselben Messschaltung oder einem anderen Sensor aufzuzeichnen, in einem Speicher zu speichern und als Eingangssignale über die veränderbare Kapazität in die Messschaltung einzuspeisen. Dadurch kann die Vorrichtung mit einer "Aufnahme und Wiedergabe"-Funktion ("record and playback") ausgestattet werden.

Dementsprechend kann im erfindungsgemässen Verfahren die auszumessende Kapazität im Wesentlichen zeitlich unverändert belassen werden, und das elektrische Steuersignal ist von einem Ausgangssignal der Messschaltung unabhängig. Das elektrische Steuersignal ist vorzugsweise ein sich zeitlich schnell änderndes, synthetisch erzeugtes und/oder vorgängig gespeichertes Signal. Diese Variante des erfindungsgemässen Verfahrens wird z. B. zum Simulieren von Messungen mit der Messschaltung, zum Testen der Messschaltung oder zum Testen von der Messschaltung nachgeschalteten Komponenten verwendet.

### AUFZÄHLUNG DER ZEICHNUNGEN

Nachfolgend wird die Erfindung anhand der schematischen Zeichnungen detailliert erläutert. Die Figuren 1 und 2 illustrieren den technischen Hintergrund der Erfindung. Die Figuren 3-6 zeigen Schaltschemata von verschiedenen Ausführungsformen der erfindungsgemässen Messschaltung.

### AUSFÜHRUNG DER ERFINDUNG

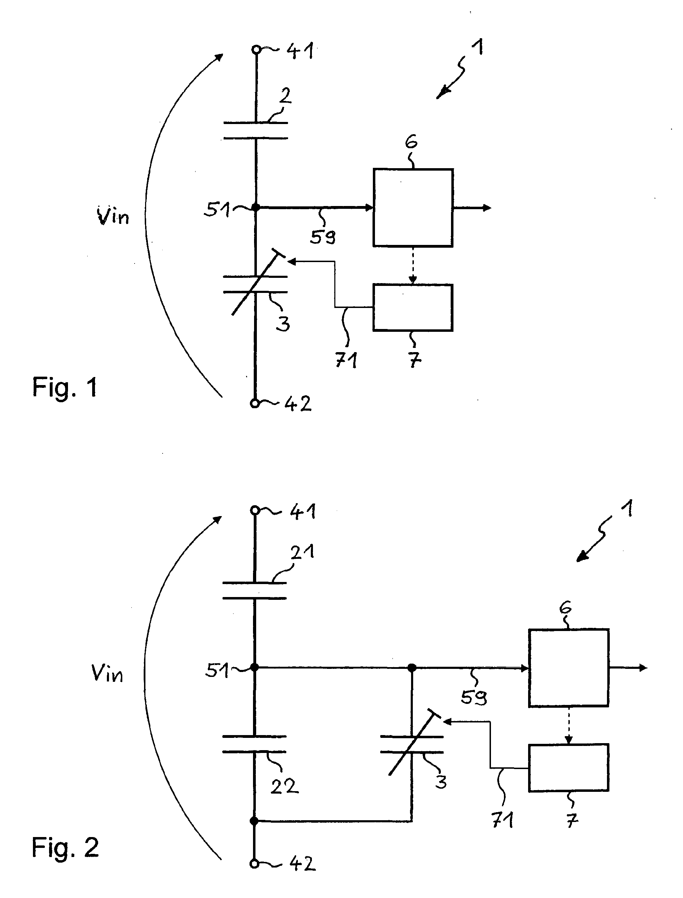

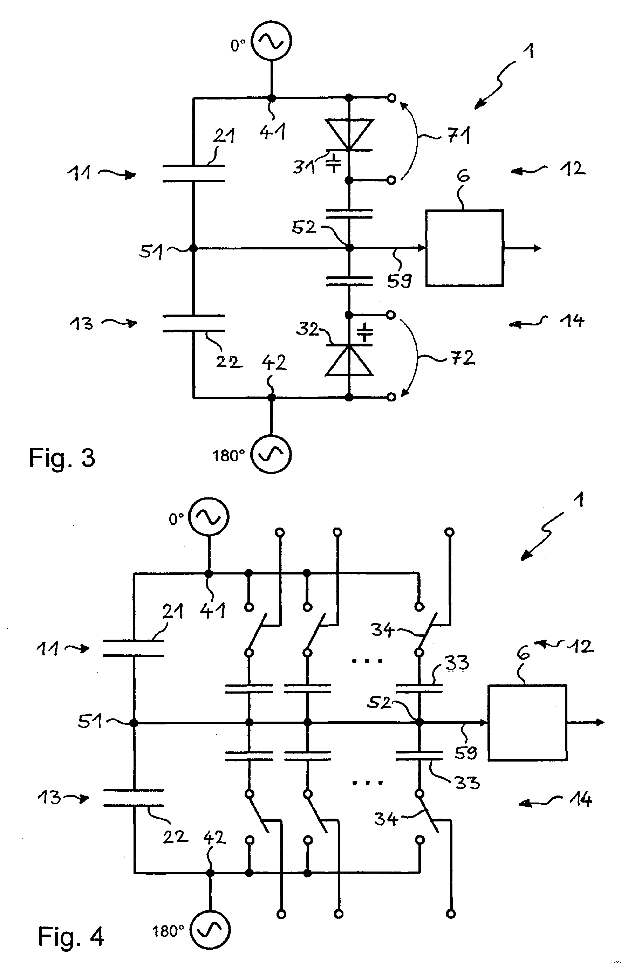

Im Schaltschema von **Figur 1** ist der technische Hintergrund der Erfindung auf einfache Weise dargestellt. Eine Messschaltung 1 beinhaltet eine auszumessende Kapazität 2 und eine durch ein elektrisches Steuersignal 71 veränderbare Kapazität 3. Auf Möglichkeiten zur Realisierung der elektrisch veränderbaren Kapazität 3 wird anlässlich der Figuren 3-5 eingegangen. Die beiden Kapazitäten 2, 3 werden hier schematisch durch Kondensatoren symbolisiert. Sie sind in diesem Ausführungsbeispiel in Serie nacheinander geschaltet. An beiden Enden der Serieschaltung befinden sich zwei Eingangsanschlüsse 41, 42 zum Anlegen eines elektrischen Wechselspannungssignals Vin, an die Messschaltung 1. Zwischen den beiden in Serie geschalteten Kapazitäten 2, 3 befindet sich ein Ausgangsanschluss 51 zur Ausgabe eines Ausgangssignals der der Messschaltung 1. Eine Ausgangsleitung 59 führt das Ausgangssignal vom Ausgangsanschluss 51 zu einer Auswerteeinheit 6 zwecks Auswertung.

Während des Abgleichs der Messschaltung 1 sollte darauf geachtet werden, dass sich die auszumessende Kapazität 2 und allenfalls andere in der Messschaltung 1 vorhandene Kapazitäten, mit Ausnahme der veränderbaren Kapazität 3, nicht ändert. Es sollten also zeitlich konstante Umgebungsbedingungen wie Temperatur und Luftfeuchtigkeit herrschen. Falls die auszumessende Kapazität 2 als Kondensator zur Aufnahme von Prüfgut ausgebildet ist, sollte sich kein Prüfgut im Kondensator befinden, oder die Kapazität des Prüfgutes sollte sich nicht verändern.

Zum Abgleich der Messschaltung 1 wird ein elektrisches Wechselspannungssignal an die Eingangsanschlüsse 41, 42 angelegt. Ein elektrisches Ausgangssignal, vorzugsweise eine Wechselspannung, wird am Ausgangsanschluss 51 abgegriffen. Die veränderbare Kapazität 3 wird durch das elektrische Steuersignal 71, vorzugsweise eine Gleichspannung, derart verändert, dass das Ausgangssignal einen bestimmten Wert annimmt, vorzugsweise Null wird. Das elektrische Steuersignal 71 kann manuell von einer Person eingestellt werden, bspw. nach der Herstellung oder beim Unterhalt der Vorrichtung, in der die Messschaltung 1 eingebaut ist. Alternativ kann das elektrische Steuersignal 71 automatisch erzeugt werden. Die automatische Erzeugung kann in einer speziell dafür vorgesehenen Steuereinheit 7 oder in der Auswerteeinheit 6 erfolgen. In Figur 1 ist schematisch eine Rückkopplung von der Auswerteeinheit 6 zur Steuereinheit 7 eingezeichnet, so dass das Steuersignal 71 vom Ausgangssignal der Messschaltung 1 abhängt. Mit einer automatischen Erzeugung des elektrischen Steuersignals 71 kann die Messschaltung 1 automatisch abgeglichen werden. Somit liegt ein geschlossener Regelkreis vor, in dem das Ausgangssignal die Regelgrösse ist, die auf den Sollwert Null geregelt werden soll, die Steuereinheit 7 als Regler wirkt und das Steuersignal 71 der Stellwert ist.

Die Messschaltung 1 von **Figur 2** unterscheidet sich von derjenigen von Figur 1 dadurch, dass sie einen parallel zur auszumessenden Kapazität 21 geschalteten Referenzkondensator 22 beinhaltet. Der Referenzkondensator 22 dient der genaueren Messung und der Kompensation von äusseren Einflüssen wie Lufttemperatur oder Luftfeuchtigkeit. Selbstverständlich können die Funktionen von Messkondensator 21 und Referenzkondensator 22 je nach Bedarf vertauscht werden, d. h. der Referenzkondensator 22 kann Messgut aufnehmen, während der Messkondensator 21 ohne Messgut ist und als Referenz dient. Auch die Messschaltung 1 von Figur 2 wird durch Anlegen eines elektrischen Steuersignals 71 an die veränderbare Kapazität 3 abgeglichen.

**Figur 3** zeigt eine erste Ausführungsform einer erfindungsgemässen Messschaltung 1. Diese ist als Kapazitätshalbmessbrücke ausgebildet. Sie beinhaltet vier Halbbrückenzweige 11-14, die jeweils mindestens eine Kapazität aufweisen. Dabei sind jeweils zwei Halbbrückenzweige 11, 12 bzw. 13, 14 parallel zueinander zu Zweigpaaren geschaltet, und die beiden Zweigpaare 11, 12 sowie 13, 14 sind in Serie zueinander geschaltet. An beiden Enden der Serieschaltung befinden sich zwei Eingangsanschlüsse 41, 42 zum Anlegen von elektrischen Eingangssignalen an die Kapazitätsmessbrücke 1. Zwei miteinander verbundene Ausgangsanschlüsse 51, 52 zur Ausgabe eines Ausgangssignals der Kapazitätshalbmessbrücke 1 befinden sich zwischen den beiden in Serie zueinander geschalteten Zweigpaaren 11, 12 bzw. 13, 14. Eine Ausgangsleitung 59 führt das Ausgangssignal von den Ausgangsanschlüssen 51, 52 zu einer Auswerteeinheit 6 zur Auswertung.

Ein zweiter Brückenzweig 12 und ein vierter Brückenzweig 14 weisen veränderbare Kapazitäten 31, 32 auf. Die veränderbaren Kapazitäten 31, 32 können mittels je einer Kapazitätsdiode (auch Kapazitäts-Variationsdiode oder Varicap genannt) realisiert sein. Dies ist ein elektronischer Baustein, dessen Kapazität mittels einer angelegten elektrischen Spannung veränderbar ist. Jede der Kapazitätsdioden 31, 32 wird durch eine analoge elektrische Steuerspannung 71, 72 gesteuert. In einer bevorzugten Ausführungsform sind die Steuerspannungen 71, 72 der beiden Kapazitätsdioden 31, 32 bezüglich einer Referenzspannung zueinander antisymmetrische Gleichspannungen, d. h. ihre Differenzen zur Referenzspannung sind betragsmässig gleich und haben entgegengesetzte Vorzeichen. Die Steuerspannungen 71, 72 können z. B. von D/A-Wandlern geliefert werden.

Die auszumessende Kapazität 21 im ersten Brückenzweig 11 ist z. B. die Kapazität eines Messkondensators, der zur Aufnahme eines (nicht dargestellten) vorzugsweise in seiner Längsrichtung bewegten länglichen Prüfgutes wie Kardenband, Vorgarn, Garn oder Gewebe geeignet ist. Ein in einem dritten Brückenzweig 13 befindlicher, mit dem Messkondensator 21 in Serie geschalteter Kondensator 22 kann als Referenzkondensator vorgesehen sein. Der Referenzkondensator 22 ermöglicht genaue, von äusseren Einflüssen wie Lufttemperatur oder Luftfeuchtigkeit nicht beeinflusste Kompensationsmessungen am Prüfgut. Der Messkondensator 21 und der Referenzkondensator 22 sollten möglichst identisch sein, um auch möglichst gleiche Kapazitäten aufzuweisen. Die beiden einander zugewandten Kondensatorplatten des Messkondensators 21 bzw. des Referenzkondensators 22, die auf demselben elektrischen Potenzial liegen, können als eine einzige Kondensatorplatte realisiert sein, wodurch bereits eine Quelle von möglichen Asymmetrien beseitigt wird.

Als Eingangssignale können in der Ausführungsform von Figur 3 z. B. ein erstes und ein zweites Wechselspannungssignal an den beiden Eingangsanschlüssen 41, 42 der Messschaltung 1 angelegt werden. Die beiden angelegten Wechselspannungssignale sind vorzugsweise zueinander antisymmetrisch, d. h. ihre Differenzen zu einer Referenzspannung sind betragsmässig gleich und um 180° gegeneinander phasenverschoben.

Ein Ausgangssignal der Messschaltung 1 wird an einer Ausgangsleitung 59 abgegriffen, die einen Halbbrückenstamm bildet, d. h. alle vier Halbbrückenzweige 11-14 in der Mitte der Kapazitätshalbmessbrücke 1 verbindet. Zum Abgleichen der Kapazitätshalbmessbrücke 1 werden die beiden Steuerspannungen derart verändert, dass das Ausgangssignal den Wert Null annimmt. Die beiden Steuerspannungen sind vorzugsweise Gleichspannungen und bezüglich einer Referenzspannung zueinander antisymmetrisch. Ist die Kapazitätsmessbrücke 1 einmal abgeglichen, so muss dafür gesorgt werden, dass im Messbetrieb die entsprechenden Steuerspannungen unverändert aufrecht erhalten bleiben. Die entsprechenden Werte können z. B. in einem digitalen Speicher gespeichert werden und über geeignete D/A-Wandler als konstante Spannungen an die Kapazitätsdioden 31, 32 abgegeben werden. Während des Abgleichs der Kapazitätshalbmessbrücke 1 sollte darauf geachtet werden, dass sich die Kapazitäten der Kapazitätshalbmessbrücke 1, mit Ausnahme der mit den elektrischen Steuersignalen 71, 72 veränderbaren Kapazitätsdioden 31, 32, nicht ändern.

Figur 3 gibt nur eines von vielen möglichen Ausführungsbeispielen einer erfindungsgemässen Messschaltung 1 mit Kapazitätsdioden 31, 32. Der Fachmann weiss, dass die Schaltungsumgebung der Kapazitätsdioden 31, 32 auch anders gestaltet werden kann. Die Kapazitätsdioden 31, 32 können durch andere Arten von veränderbaren Kapazitäten ersetzt werden. Prinzipiell würde eine einzige veränderbare Kapazität 31, z. B. diejenige im zweiten Halbbrückenzweig 12, genügen, um die Kapazitätshalbmessbrücke 1 abzugleichen. Aus Symmetriegründen ist die Ausführung mit zwei veränderbaren Kapazitäten 31, 32 gemäss Figur 3 für die meisten Anwendungen vorteilhafter.

In einer Weiterbildung dieser Ausführungsform der erfindungsgemässen Messschaltung 1 können der Messkondensator 21 im ersten Halbbrückenzweig 11 und der Referenzkondensator 21 im dritten Halbbrückenzweig 13, die zusammen als eine Messeinheit angesehen werden können, mehrfach vorhanden sein. Die weiteren derartigen Messeinheiten sind dann parallel zur in Figur 3 eingezeichneten ersten Messeinheit geschaltet. Sie beinhalten vorzugsweise Kondensatoren mit unterschiedlichen Kapazitäten. Diese können zur Ausmessung von unterschiedlichem Prüfgut, z. B. von verschieden dicken Garnen, verwendet werden. Die Unterscheidung von Mess- und Referenzkondensator dient in dieser Schrift eher einer eindeutigen Namensgebung als einer starren Zuweisung von Funktionen, denn in den meisten Fällen kann ein Messkondensator auch als Referenzkondensator dienen und umgekehrt. Sind in einer Messschaltung mehrere Mess- und Referenzkondensatoren vorhanden, so dient in der Regel bei einer Messung einer der Kondensatoren als Messkondensator und die übrigen Kondensatoren als Referenzkondensatoren.

Eine zweite Ausführungsform einer als Kapazitätshalbmessbrücke ausgebildeten erfindungsgemässen Messschaltung 1 ist in Figur 4 dargestellt. Diejenigen Elemente, welche zur ersten Ausführungsform von Figur 3 analog sind, sind in Figur 4 mit denselben Bezugszeichen bezeichnet und werden hier nicht mehr erläutert. Die zweite Ausführungsform unterscheidet sich von der ersten durch die Realisierung der veränderbaren Kapazität des zweiten Halbbrückenzweiges 12 und vierten Halbbrückenzweiges 14. Der zweite Halbbrückenzweig 12 und der vierte Halbbrückenzweig 14 enthalten jeweils eine Mehrzahl von zueinander parallel geschalteten, an sich nicht veränderbaren Abgleichkondensatoren 33, die mit entsprechenden elektrischen Steuersignalen zu- und wegschaltbar sind. Dadurch haben der zweite Halbbrückenzweig 12 und der vierte Halbbrückenzweig 14 jeweils eine durch die Steuersignale veränderbare Kapazität. Die Abgleichkondensatoren 33 können untereinander identisch oder verschieden sein. Um einen möglichst grossen Bereich abzudecken, in dem abgeglichen werde kann, werden die Abgleichkondensatoren 33 vorzugsweise verschieden voneinander gewählt. Es könnte z. B. jeder nachfolgende Abgleichkondensator 33 eine halb so grosse Kapazität aufweisen wie sein vorangehender Nachbar. Die Anordnungen der Abgleichkondensatoren 33 im zweiten Halbbrückenzweig 12 und im vierten Halbbrückenzweig 14 sind vorzugsweise zueinander symmetrisch.

Jeder einzelne Abgleichkondensator 33 kann mittels eines ihm zugeordneten Schalters 34 zu- oder weggeschaltet werden. Die Schalter 34 werden von entsprechenden digitalen Steuersignalen geschaltet. Die Steuersignale können von einem an sich bekannten digitalen Treiberbaustein geliefert werden. Auch in diesem Ausführungsbeispiel muss die für den Abgleich benötigte Schalterstellung für den Messbetrieb gespeichert und aufrecht erhalten werden.

Es gibt verschiedene, an sich bekannte Möglichkeiten, die Schalter 34 zu realisieren. Bei Verwendung eines zusätzlichen Serie-Kondensators ist es auch möglich, die Abgleichkondensatoren 33 parallel zu den Schaltern 34 zu legen.

Die Mehrzahl von zueinander parallel geschalteten Abgleichkondensatoren 33 in jeweils einem Halbbrückenzweig 12, 14 kann in einem einzigen Baustein integriert sein. Die Abgleichkondensatoren 33 sind dann vorzugsweise als mikro-elektromechanisches System (englisch micro-electro-mechanical system, MEMS) ausgeführt. Eine integrierte MEMS-Zeilenanrodnung von zuschaltbaren Kapazitäten wird z. B. unter der Bezeichnung WSC002L von der Firma WiSpry, Inc, Irvine, CA, USA, angeboten.

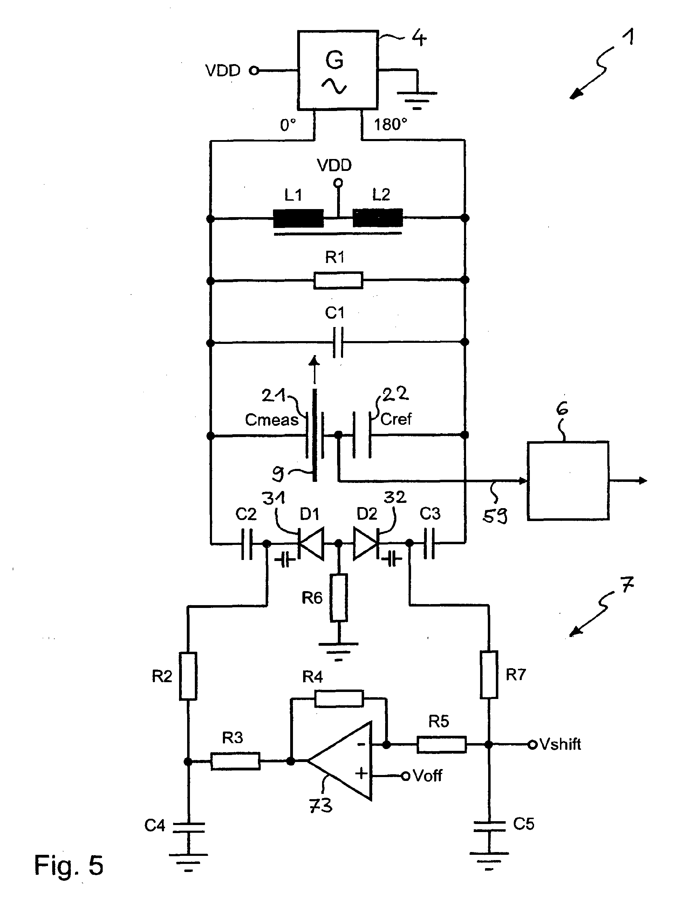

Figur 5 zeigt eine weitere Ausführungsform der erfindungsgemässen Messschaltung 1 mit veränderbaren Kapazitäten 31, 32. Die veränderbaren Kapazitäten 31, 23 sind hier mit den Schaltungssymbolen für Kapazitätsdioden dargestellt, könnten jedoch auch anders realisiert sein. Eine derartige Messschaltung 1 wird vorzugsweise zur kapazitiven Untersuchung eines bewegten länglichen Prüfgutes 9, z. B. eines Garns, eingesetzt. Das Prüfgut 9 durchläuft einen Messspalt, der durch zwei vorzugsweise ebene Elektroden eines Messkondensators 21 gebildet wird. Zum Messkondensator 21 in Serie geschaltet ist ein Referenzkondensator 22, der kein Messgut aufnimmt. Messkondensator 21 und Referenzkondensator 22 sind Teile eines LC-Schwingkreises 11, der zwei durch einen gemeinsamen Kern gekoppelte Spulen L1, L2, einen Widerstand R1 und einen Kondensator C1 beinhaltet. Der Schwingkreis 11 wird von einem Oszillator 4 angeregt. Der Oszillator 4 erhält eine Speisespannung VDD und erzeugt in den beiden Ästen des Schwingkreises 11 zwei im Wesentlichen sinusförmige Wechselspannungssignale, die gegeneinander um 180° phasenverschoben sind.

Zum Abgleichen beinhaltet die Messschaltung 1 zwei veränderbare Kapazitäten 31, 32 und eine Steuerschaltung 7 zum Ansteuern der veränderbaren Kapazitäten 31, 32. Der Kern der Steuerschaltung 7 ist ein Verstärker 73, an dessen einem Eingang eine Offsetspannung Voff von bspw. 2.5 V angelegt wird. Ferner wird an die Steuerschaltung 7 eine Abgleichspannung Vshift, die bspw. zwischen 0 und 4 V betragen kann, angelegt. Die Steuerschaltung 7 ist derart ausgebildet, dass die an den beiden veränderbaren Kapazitäten 31, 32 anliegenden Steuerspannungen zueinander bezüglich einer Referenzspannung antisymmetrische Gleichspannungen sind, d. h. ihre Differenzen zur Referenzspannung sind betragsmässig gleich und haben entgegengesetzte Vorzeichen. Dabei wird im Wesentlichen die Referenzspannung durch die Offsetspannung Voff und die Differenzen durch die Abgleichspannung Vshift eingestellt. Bezüglich der in der Steuerschaltung 7 eingesetzten Widerstände gilt vorzugsweise: R3 << R2 = R6 = R7, R4 = R5.

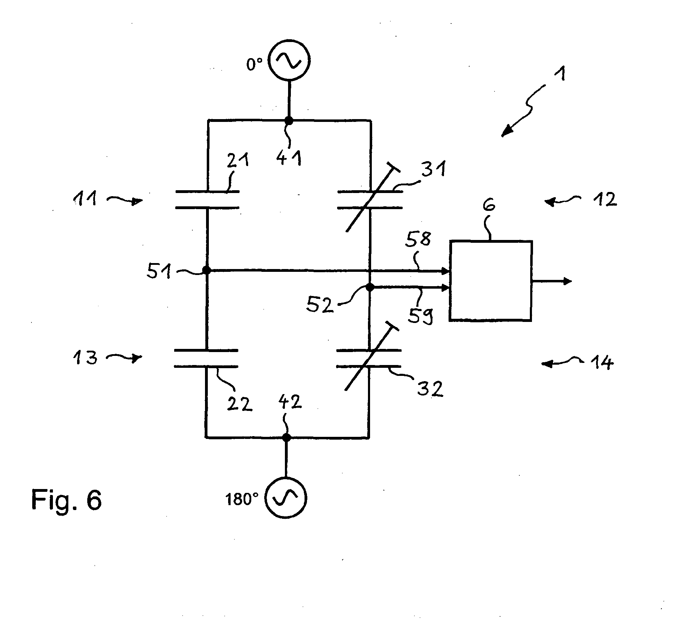

Eine Weiterbildung der Messschaltung von Figur 3 ist die Messschaltung 1, wie sie in Figur 6 dargestellt und nachfolgend beschrieben ist. Wiederum sind diejenigen Elemente, welche zur Ausführungsform von Figur 3 analog sind, in Figur 6 mit denselben Bezugszeichen bezeichnet und werden hier nicht mehr erläutert. Der Hauptunterschied zur Ausführungsform von Figur 3 besteht darin, dass die vier Brückenzweige 11-14 nicht durch einen Brückenstamm miteinander verbunden sind. Somit handelt es sich hier um eine Kapazitätsvollmessbrücke, im Gegensatz zur Kapazitätshalbmessbrücke von Figur 3. In der Kapazitätsmessbrücke 1 gemäss Figur 6 werden zwei Ausgangssignale abgegriffen: ein erstes am Ausgangsanschluss 51 an der Verbindungsstelle zwischen dem ersten Brückenzweig 11 und dem dritten Brückenzweig 13, und ein zweites am Ausgangsanschluss 52 an der Verbindungsstelle zwischen dem zweiten Brückenzweig 12 und dem vierten Brückenzweig 14. Das erste und das zweite Ausgangssignal werden über eine erste Ausgangsleitung 58 bzw. eine zweite Ausgangsleitung 59 der Auswerteeinheit 6 zugeführt. Die Auswerteeinheit 6 ermittelt die Differenz zwischen den beiden Ausgangssignalen; es handelt sich hier daher um ein differenzielles Messverfahren. Die Messbrücke 1 ist dann abgeglichen, wenn die besagte Differenz null ist. Zum Abgleich der Messbrücke 1 dienen wiederum eine oder zwei veränderbare Kapazitäten 31, 32 im zweiten Brückenzweig 12 bzw. im vierten Brückenzweig 14. Diese können als Kapazitätsdioden, als MEMS-Kapazitäten oder auf eine andere Weise ausgeführt sein.

Selbstverständlich ist die vorliegende Erfindung nicht auf die oben diskutierten Ausführungsformen beschränkt. Bei Kenntnis der Erfindung wird der Fachmann weitere Varianten herleiten können, die auch zum Gegenstand der vorliegenden Erfindung gehören. Solche Varianten können z. B. Kombinationen der oben diskutierten Ausführungsformen sein. So kann z. B. die Kapazitätshalbbrücke in der Messschaltung 1 von Figur 5 durch eine Kapazitätsvollbrücke gemäss Figur 6 ersetzt werden. Dem Fachmann sind an sich viele elektrische Komponenten bekannt, deren Kapazität durch ein elektrisches Signal veränderbar ist.

### BEZUGSZEICHENLISTE

- 1: Messschaltung
- 11-14: Brückenzweigen

- 2: auszumessende Kapazität
- 21: Messkondensator
- 22: Referenzkondensator

- 3: veränderbare Kapazität
- 31, 32: Kapazitätsdioden
- 33: Abgleichkondensatoren
- 34: Schalter

- 4: Oszillator
- 41, 42: Eingangsanschlüsse

- 51, 52: Ausgangsanschlüsse
- 58, 59: Ausgangsleitungen

- 6: Auswerteeinheit

- 7: Steuerschaltung
- 71, 72: Steuersignale
- 73: Verstärker

- 9: Prüfgut

## Patentansprüche

1. Vorrichtung zur kapazitiven Untersuchung eines bewegten länglichen Prüfgutes (9), beinhaltend eine kapazitive Messschaltung (1) mit einem Messkondensator (21) zur Aufnahme des Prüfgutes (9), wobei
die Messschaltung (1) als Kapazitätsmessbrücke mit vier Brückenzweigen (11-14) ausgebildet ist,
ein erster Brückenzweig (11) ein Messzweig ist, der den Messkondensator (21) beinhaltet, und
ein zweiter Brückenzweig (12) ein Bauteil (31) mit einer durch mindestens ein elektrisches Steuersignal (71) veränderbaren Kapazität beinhaltet,
**dadurch gekennzeichnet,**
**dass** ein vierter Brückenzweig (14) ein Bauteil (32) mit einer durch mindestens ein elektrisches Steuersignal (72) veränderbaren Kapazität beinhaltet und
ein dritter Brückenzweig (13) einen Referenzkondensator (22) beinhaltet.

2. Vorrichtung (1) nach Anspruch 1, wobei das mindestens eine Bauteil (31, 32) ein Bauteil aus der folgenden Gruppe ist: Kapazitätsdiode (31, 32), andere Diode, eine Mehrzahl von zueinander parallel geschalteten Abgleichkondensatoren (33), die mit entsprechenden elektrischen Steuersignalen zu- und wegschaltbar sind, insbesondere MEMS-Kondensatoren, in Sperrrichtung betriebener Transistor, Metall-Oxid-Halbleiter-Feldeffekttransistor, mikro-elektromechanischer Kondensator, Kondensator, dessen Elektrodenabstand mittels eines Piezoelementes veränderbar ist.

3. Vorrichtung (1) nach einem der vorangehenden Ansprüche, wobei die Kapazitäten der zwei Bauteile (31, 32) im Wesentlichen antisymmetrisch zueinander veränderbar sind.

4. Vorrichtung nach einem der vorangehenden Ansprüche, wobei die Messschaltung (1) als Kapazitätsvollmessbrücke mit vier Brückenzweigen (11-14), die nicht durch einen Brückenstamm miteinander verbunden sind, ausgebildet ist.

5. Vorrichtung nach Anspruch 4, wobei eine Auswerteeinheit (6) zur Ermittlung einer Differenz zwischen
einem ersten Ausgangssignal, das an einer Verbindungsstelle (51) zwischen dem ersten Brückenzweig (11) und dem dritten Brückenzweig (13) abgegriffen wird, und
einem zweiten Ausgangssignal, das an einer Verbindungsstelle (52) zwischen dem zweiten Brückenzweig (12) und dem vierten Brückenzweig (14) abgegriffen wird,
vorgesehen ist.

6. Vorrichtung nach einem der vorangehenden Ansprüche, ferner beinhaltend Mittel (4) zum Anlegen eines Wechselspannungssignals (Vin) an die Messschaltung (1) zwecks Erzeugung eines elektrischen Wechselfeldes im Messkondensator (21), und
eine Durchgangsöffnung für das Prüfgut (9) im Messkondensator (21), welche Durchgangsöffnung vom elektrischen Wechselfeld beaufschlagbar ist.

7. Verfahren zum Betreiben einer Messschaltung (1) in der Vorrichtung nach einem der vorangehenden Ansprüche,
**dadurch gekennzeichnet,**
**dass** die zwei veränderbaren Kapazitäten (31, 32) durch mindestens ein elektrisches Steuersignal (71, 72) verändert werden.

8. Verfahren nach Anspruch 7, wobei die zwei veränderbaren Kapazitäten durch Anlegen des mindestens einen Steuersignals (71, 72) an jeweils eine Kapazitätsdiode (31, 32) verändert werden.

9. Verfahren nach Anspruch 7 oder 8, wobei die zwei veränderbaren Kapazitäten jeweils durch Zu- und/oder Wegschalten einer Mehrzahl von zueinander parallel geschalteten Abgleichkondensatoren (33) mit entsprechenden elektrischen Steuersignalen verändert werden.

10. Verfahren nach einem der Ansprüche 7-9, wobei die Kapazitäten (31, 32) im Wesentlichen antisymmetrisch zueinander verändert werden.

11. Verfahren nach einem der Ansprüche 7-10, wobei
der Messkondensator (21) im Wesentlichen zeitlich unverändert belassen wird,
ein elektrisches Wechselspannungssignal (Vin) an die Messschaltung (1) angelegt wird,
ein elektrisches Ausgangssignal der Messschaltung (1) abgegriffen wird und
das mindestens eine elektrische Steuersignal (71, 72) durch das Ausgangssignal beeinflusst wird.

12. Verfahren nach Anspruch 11, wobei die Beeinflussung des mindestens einen elektrischen Steuersignals (71, 72) durch das Ausgangssignal automatisch in einem geschlossenen Regelkreis erfolgt.

13. Anwendung des Verfahrens nach Anspruch 11 oder 12 zum Abgleichen der Messschaltung (1).

14. Verfahren nach einem der Ansprüche 7-10, wobei
der Messkondensator (21) im Wesentlichen zeitlich unverändert belassen wird und das mindestens eine elektrische Steuersignal von einem Ausgangssignal der Messschaltung (1) unabhängig ist.

15. Verfahren nach Anspruch 14, wobei das mindestens eine elektrische Steuersignal ein sich zeitlich schnell änderndes, synthetisch erzeugtes und/oder vorgängig gespeichertes Signal ist.

16. Anwendung des Verfahrens nach Anspruch 14 oder 15 zum Simulieren von Messungen mit der Messschaltung (1), zum Testen der Messschaltung (1) oder zum Testen von der Messschaltung (1) nachgeschalteten Komponenten.

## Claims

1. An apparatus for the capacitive examination of a moved elongated test material (9), containing a capacitive measuring circuit (1) with a measuring capacitor (21) for accommodating the test material (9), wherein
the measuring circuit (1) is arranged as a capacitance measuring bridge with four bridge arms (11 to 14),
a first bridge arm (11) is a measuring arm which contains the measuring capacitor (21), and
a second bridge arm (12) contains a component (31) with a capacitance which is changeable by at least one electric control signal (71),
**characterized in that**
a fourth bridge arm (14) contains a component (32) with a capacitance which is changeable by at least one electric control signal (72) and
a third bridge arm (13) contains a reference capacitor (22).

2. The apparatus according to claim 1, wherein the at least one component (31, 32) is a component from the following group: variable-capacitance diode (31, 32), other diode, a plurality of balancing capacitors (33) which are connected in parallel with respect to each other and which can be connected and disconnected with respective electric control signals, especially MEMS capacitors, a reverse-biased transistor, metal-oxide semiconductor field effect transistor, micro-electro-mechanical capacitor, capacitor whose electrode distance is changeable by means of a piezo element.

3. The apparatus (1) according to one of the preceding claims, wherein the capacitances of the two components (31, 32) are changeable in a substantially anti-symmetrical way with respect to each other.

4. The apparatus according to one of the preceding claims, wherein the measuring circuit (1) is arranged as a full capacitance measuring bridge with four bridge arms (11 to 14) which are not connected with each other by a bridge stem.

5. The apparatus according to claim 4, wherein an evaluation unit (6) is provided for determining a difference between
a first output signal which is tapped from a connection point (51) between the first bridge arm (11) and the third bridge arm (13), and
a second output signal which is tapped from a connection point (52) between the second bridge arm (12) and the fourth bridge arm (14).

6. The apparatus according to one of the preceding claims, further containing means (4) for applying an alternating voltage signal (Vin) to the measuring circuit (1) for the purpose of generating an alternating electrical field in the measuring capacitor (21), and
a through-opening for the test material (9) in the measuring capacitor (21), which through-opening is subjectable to the alternating electrical field.

7. A method for operating a measuring circuit (1) in the apparatus according to one of the preceding claims,
**characterized in that**
the variable capacitances (31, 32) are changed by at least one electric control signal (71, 72).

8. The method according to claim 7, wherein the two variable capacitances are changed by applying the at least one control signal (71, 72) to one variable-capacitance diode (31, 32) each.

9. The method according to claim 7 or 8, wherein the two variable capacitances are changed by connecting and/or disconnecting a plurality of balancing capacitors (33) with respective electric control signals, which balancing capacitors are connected in parallel with respect to each other.

10. The method according to one of the claims 7 to 9, wherein the capacitances (31, 32) are changed in a substantially anti-symmetrical way with respect to each other.

11. The method according to one of the claims 7 to 10, wherein
the measuring capacitor (21) is left in a substantially temporally unchanged manner, an electric alternating voltage signal (Vin) is applied to the measuring circuit (1),
an electric output signal of the measuring circuit (1) is tapped, and
the at least one electric control signal (71, 72) is influenced by the output signal.

12. The method according to claim 11, wherein the influence on the at least one electric control signal (71, 72) is provided automatically by the output signal in a closed control loop.

13. Application of the method according to claim 11 or 12 for balancing the measuring circuit (1).

14. The method according to one of the claims 7 to 10, wherein
the measuring capacitor (21) is left in a substantially temporally unchanged way and the at least one electric control signal is independent of an output signal of the measuring circuit (1).

15. The method according to claim 14, wherein the at least one electric control signal is a temporally rapidly changing, synthetically generated and/or previously stored signal.

16. Application of the method according to claim 14 or 15 for simulating measurements with the measuring circuit (1), for testing the measuring circuit (1) or for testing components connected in series with the measuring circuit (1).

## Revendications

1. Dispositif pour l'inspection capacitive d'un échantillon allongé (9) en mouvement, comprenant un circuit de mesure capacitif (1) avec un condensateur de mesure (21) destiné à recevoir l'échantillon (9), dans lequel
le circuit de mesure (1) est conçu comme un pont de mesure capacitif avec quatre branches de pont (11-14),
une première branche de pont (11) est une branche de pont contenant le condensateur de mesure (21), et
une deuxième branche de pont (12) contient un composant (31) ayant une capacité modifiable par au moins un signal de commande électrique (71),
**caractérisé en ce**
**qu'**une quatrième branche de pont (14) contient un composant (32) ayant une capacité modifiable par au moins un signal de commande électrique (72) et
une troisième branche de pont (13) contient un condensateur de référence (22).

2. Dispositif (1) selon la revendication 1, dans lequel l'au moins un composant (31, 32) est un composant faisant partie du groupe suivant : diode de capacité (31, 32), autre diode, une pluralité de condensateurs d'égalisation (33) montés en parallèle, qui peuvent être mis en circuit et hors circuit par des signaux de commande électriques correspondants, en particulier des condensateurs MEMS, un transistor excité dans le sens bloquant, un transistor à effet de champ à semi-conducteur métal-oxyde, un condensateur micro-électromécanique, un condensateur dont l'écart entre les électrodes est modifiable au moyen d'un élément piézoélectrique.

3. Dispositif (1) selon l'une des revendications précédentes, dans lequel les capacités des deux composants (31, 32) sont modifiables de manière sensiblement antisymétrique l'une par rapport à l'autre.

4. Dispositif selon l'une des revendications précédentes, dans lequel le circuit de mesure (1) est construit comme un pont capacitif intégral avec quatre branches de porte (11-14) qui ne sont pas reliées par un corps de pont.

5. Dispositif selon la revendication 4, dans lequel est prévue une unité d'analyse (6) pour déterminer la différence entre
un premier signal de sortie capté à un point de liaison (51) entre la première branche de pont (11) et la troisième branche de pont (13) et
un deuxième signal de sortie capté à un point de liaison (52) entre la deuxième branche de pont (12) et la quatrième branche de pont (14).

6. Dispositif selon l'une des revendications précédentes, contenant en outre des moyens (4) pour appliquer une tension de courant alternatif (Vin) au circuit de mesure (1) en vue de produire un champ alternatif électrique dans le condensateur de mesure (21), et
une ouverture de passage pour l'échantillon (9) dans le condensateur de mesure (21), laquelle ouverture de passage peut être exposée au champ alternatif électrique.

7. Procédé pour faire fonctionner un circuit de mesure (1) dans le dispositif selon l'une des revendications précédentes,
**caractérisé en ce que**
les deux capacités modifiables (31, 32) sont modifiées par au moins un signal de commande électrique (71, 72).

8. Procédé selon la revendication 7, dans lequel les deux capacités modifiables sont modifiées par l'application de l'au moins un signal de commande (71, 72) à au moins une diode à capacité variable (31, 32).

9. Procédé selon la revendication 7 ou 8, dans lequel les deux capacités modifiables sont modifiées chacune par la mise en circuit et/ou hors circuit d'une pluralité de condensateurs d'égalisation (33) montés en parallèle les uns avec les autres par des signaux de commande électriques correspondants.

10. Procédé selon l'une des revendications 7 à 9, dans lequel les capacités (31, 32) sont modifiées de façon sensiblement antisymétrique les unes par rapport aux autres.

11. Procédé selon l'une des revendications 7 à 10, dans lequel
le condensateur de mesure (21) est laissé sensiblement inchangé dans le temps,
un signal de tension alternative électrique (Vin) est appliqué au circuit de mesure (1),
un signal de sortie électrique du circuit de mesure (1) est capté et
l'au moins un signal de commande électrique (71, 72) est influencé par le signal de sortie.

12. Procédé selon la revendication 11, dans lequel l'au moins un signal de commande électrique (71, 72) est influencé par le signal de sortie de façon automatique, dans un circuit de régulation fermé.

13. Mise en oeuvre du procédé selon la revendication 11 ou 12 pour égaliser le circuit de mesure (1).

14. Procédé selon l'une des revendications 7 à 10, dans lequel
le condensateur de mesure (21) est laissé sensiblement inchangé dans le temps et
l'au moins un signal de commande électrique est indépendant d'un signal de sortie du circuit de mesure (1).

15. Procédé selon la revendication 14, dans lequel l'au moins un signal de commande électrique est un signal se modifiant rapidement dans le temps, produit par synthèse et/ou enregistré en mémoire au préalable.

16. Mise en oeuvre du procédé selon la revendication 14 ou 15 pour simuler des mesures avec le circuit de mesure (1), pour tester le circuit de mesure (1) ou pour tester des composants montés en aval du circuit de mesure (1).
